# EUROPEAN PATENT APPLICATION

(11) **EP 1 090 638 A1**
(43) Date of publication of application: **11.04.2001**
(21) Application number: 99120169.0
(22) Date of filing: 08.10.1999
(51) Int. Cl.: A61K 31/439, A61P 25/00

(54) **Use of (R)-zacopride or a salt thereof for the manufacture of a medicament for the treatment of sleep apnea**

(71) Applicant: SANOFI-SYNTHELABO, 75013 Paris (FR)
(72) Inventor: Depoortere, Henri, 78720 Cernay-La-Ville (FR)
(74) Representative: Thouret-Lemaitre, Elisabeth

(57) **Abstract**

The present invention relates to the use of (*R*)-zacopride or a salt thereof in the manufacture of drugs intended for the treatment of sleep apnea.

## Description

The present invention relates to the use of (*R*)-zacopride or a pharmaceutically acceptable salts thereof for the manufacture of drugs intended for the treatment of sleep apnea.

Obstructuve sleep apnea (OSA), as defined by the presence of repetitive upper airway obstruction during sleep, has been identified in as many as 24% of working adult men, and 9% of similar women (Young et al., The occurence of sleep-disordered breathing among middle-aged adults., New England, *J. Med.* 328 : 1230-1235, 1993), with peak prevalence in the sixth decade (Gislason et al., Prevalence of sleep apnea syndrome among Swedish men - an epidemiological study., *J. Clin. Epidemiol* 41 : 571-576, 1988). Central sleep apnea (CSA), defined as transient and repetitive cessation of respiratory effort during sleep, is less prevalent as syndrome than OSA, but can be identified in a wide spectrum of patients with medical, neurologic, and neuromuscular disorders associated with diurnal alveolar hypoventilation or periodic breathing (Bradley et al., Central sleep apnea, *Clin. Chest. Med.* 13 : 493-505, 1992).
Although they have been described separately, OSA and CSA may in fact reflect similar pathological mechanisms. Most patients with "central" or "obstructive" sleep apnea syndrome in fact present a combination of central, mixed and obstructive apneas during a single sleep period.

The current standard of therapy for most patients with sleep apnea is very poor. Patients suffering from sleep apnea may be treated with an apparatus known as a continuous positive airway pressure (CPAP) but compliance problems appear frequently. A variety of upper airway and craniofacial surgeries have also been attempted for treatment of sleep apnea syndrome but have not proven effective in most cases. Moreover no satisfactory pharmacotherapy exists for the time being.

The lack of adequate long term therapy for sleep apnea syndrome is then a significant public health concern because patients with untreated apnea are at increased risk for cardiovascular morbidity and mortality.

It has now been found that (*R*)-zacopride or its pharamceutically acceptable salts and particularly (*R*)-zacopride hydrochloride have activity in significantly decreasing sleep apnea syndrome.

The invention therefore further relates to a method of treating sleep apnea by administering to a patient in need of such a treatment a therapeutically effective amount of (*R*)-zacopride or a salt thereof and particularly (*R*)-zacopride hydrochloride.

(*R*)-zacopride or (*R*)-*N*-[1-azabicyclo(2,2,2)oct-3-yl]-2-méthoxy-4-amino-5-chlorobenzamide is known for its antiemetic properties.
Its chemical synthesis is described in EP 099 789.

The active substance according to the invention can be administred to patients in a variety of pharmaceutical forms well-known in the art and particularly in the form of compositions formulated for administration by the oral, injectable, transdermal or rectal route.

For oral administration, which is preferable for reasons of patient convenience, said compositions can take the form of tablets, dragees or capsules prepared by the conventional techniques using known carriers and excipients, such as binding agents, fillers, lubricants and desintegration agents; they can also be in form of solutions, syrups or suspensions.
For administration by the injectable route, the compositions according to the invention may be in the form of injectable solutions, suspensions or emulsions containing an acceptable oily or aqueous liquid carrier. For transdermal administration, the composition can take the form of a patch wherein the drug can be emcompassed in a gel, solution, ointment or cream.
For rectal administration, the compositions may be in the form of suppositories containing the conventional bases for suppositories.

The percentage of active compound in such compositions may be varied so that a suitable dosage is obtained. The dosage administered to a particular patient is determined by the clinician according to the mode of administration, the age and weight of the patient and the patients response. Unit dosage forms may be administred in a single dose or in multiple divided doses to provide the appropriate daily dosage.

(*R*)-zacopride or salts thereof can be administered at daily dosage of up to 1 mg/kg of (*R*)-zacopride base.

The daily dosage of (*R*)-zacopride base can range from about 3 to 60 mg, preferably from about 10 to 30 mg.

The following examples relating to pharmacological data and a galenic formulation illustrate the present invention.

### Example 1

The efficacy of (*R*)-zacopride in treating sleep apnea was demonstrated in a rat model of sleep disordered breathing described among all in M.Radulovski et al., Serotonin 5-HT₃-receptor Antagonist GR 38032F Suppresses Sleep Apneas in Rats, *Sleep,* Vol. 21, No.2, 1998.

### METHODS

Seven adult (200-500 gram) male Sprague-Dawley rats were anesthetized (ketamine, 80 mg/kg and acetylpromazine, 2 mg/kg IP) and a surgical incision of the scalp was made to allow bilateral implantation of stainless steel screws into frontal and parietal bones of the skull for EEG recording. Bilateral wire electrodes were placed into the nuchal muscles for EMG recording. The EEG and EMG leads were soldered to a miniature connector and fixed to the skull with cranioplastic cement. The skin was then sutured and the rats were allowed at least seven days for surgical recovery.
Throughout the surgical and experimental period, rats were maintained on 12 hour light and 12 hour dark cycle in a fixed environment at 20°C with 40% humidity. Food and water were available ad libitum.
Respirations were recorded by placing each rat inside a single chamber plethysmograph (PLYUN1R/U; Buxco Electonics,Sharon, CT; dimensions 6"W X 10"L X 6"H). Thermal fluctuactions associated with tidal respiration induce changes in pressure within the plethysmograph which, under appropriate conditions, are proportional to tidal volume. Pleythysmograph pressure was transduced using a Validyne DP45-14 differential pressure transducer (± 2 cm H₂O). Plethysmograph pressure was referenced to a low-pass filtered (5 second time constant) version of itself in order to minimize the effects of any drift in temperature or ambient pressure during a recording. To minimize any possible artifact related to asymmetry or non-uniformity of pressure within the rectangular chamber, the transducer was mounted to and centered on the lid of the plethysmograph. EEG and EMG activity was carried from the connector plug on the rat head by a cable and passed through a sealed port in the plethysmograph. EEG, EMG, and respirations were continuously digitized (100 per second), displayed on computer monitor (Experimenter's Workbench; Datawave Systems, Longmont CO) and stored to disk. All signals were low-pass filtered (50 Hz corner frequency, 6-pole Butterworth filter) to prevent aliasing.
All polygraphic recordings were six hours in length and were made between 10:00 and 16:00. Each rat was recorded on 4 occasions in random order, with each recording for an individual animal separated by at least 3 days. Fifteen minutes prior to each recording (09:45) each animal received a 1ml/kg intraperitoneal bolus injection of one of 4 injectates: saline (control) or 0.05 mg/kg, 1.0 mg/kg or 10.0 mg/kg of (*R*)-zacopride hydrochloride. The study was a repeated measures balanced design, such that each animal was recorded exactly once for each injectate.
Polygraphic recordings of sleep and wakefulness were assessed by computer algorithm using the bi-frontal EEG and nuchal EMG signals on 10 second epochs. This software discriminates wakefulness (W) as a high frequency, low amplitude EEG with concomitant high EMG tone, non-rapid eye movement (NREM) sleep by increased spindles and theta EEG together with decreased EMG, and rapid eye movement (REM) sleep by a low ratio of delta to theta band EEG activity and an absence of EMG tone.
Throughout each 6 hour recording, each breath was detected by an adaptive threshold algorithm (DataWave Systems, Longmont, CO) to detect and measure respiratory rate and inspiratory minute ventilation (VI) for each breath. Normalized VI (NVI) was also computed by dividing the value for each breath by the mean value recorded during wakefulness in control recordings. This normalized measure falicitated examining the effects of sleep and injectate on respiratory pattern. Sleep apneas, defined as cessation of respiratory effort for at least 2.5 seconds, were scored for each recording session and were assiociated with the stage in which they occured; W, NREM or REM sleep. The duration requirement of 2.5 seconds was arbitrarily chosen, but reflects at least 2 "missed" breaths. The events detected represent central apneas, because decreased ventilation associated with obstructed or occluded airways would generate an increased plethysmographic signal, rather than a pause. Apnea index (AI), defined as apneas per hour in stage were separately determined for NREM and REM sleep. The major effects of recording hour, sleep state and injectate were assessed using separate one way analyses of variance (ANOVAs) with repeated measures. Interaction terms were evaluated using multi-way ANOVAs. Multiple comparisons between means were controlled using the Fisher PLSD.

### FIGURES

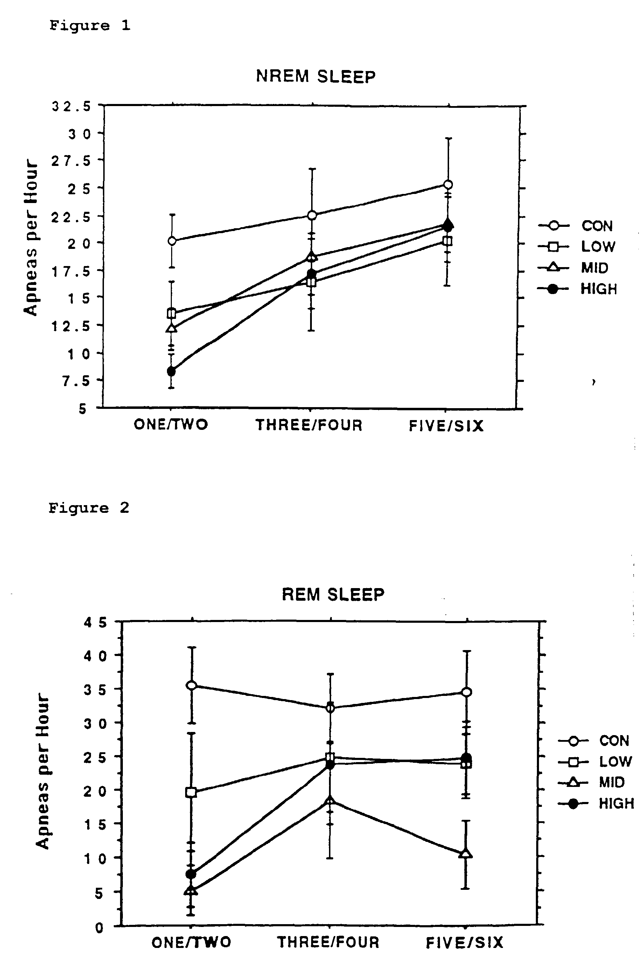

**Figure 1** represents the temporal effects of (*R*)-zacopride on apnea expression during NREM sleep.
Ordinate shows apneas per hour of NREM sleep; abscissa delineates time after injection, grouped as hours one/two, three/four and five/six. Doses of (*R*)-zacopride administred were: CON = control (saline), LOW = 0.5 mg/kg, MID = 1.0 mg/kg and HIGH = 10.0 mg/kg. Values reflect group mean ± standard error for 7 animals. Apneas scored as respiratory pauses > 2.5 seconds in duration corresponds to at least 3 "missed breaths". This criterion is analogous to a 10 second duration requirement for human apnea.

**Figure 2** represents the effects of (*R*)-zacopride on apnea expression during REM sleep. Data for REM sleep are presented in the format of Figure 1 above.

### RESULTS

(*R*)-zacopride hydrochloride produced a 28% decrease in apnea expression during NREM sleep (p=0.098) which was equivalent at all doses tested. During REM sleep, apneas were suppressed by 47% versus control by the MID dose of active compound (p=0.04), with the low and high doses being less effective (18% and 8% apnea suppression, respectively - not statistically significant).

These results show clearly the potency of (*R*)-zacopride in treating sleep apnea.

### Example 2 Oral formulation

Tablets containing (*R*)-zacopride hydrochloride dosed at 5 mg and 60 mg (*R*)-zacopride base

These are prepared with the compositions below:

| | 3 mg tablets | | 60 mg tablets | |
|---|---|---|---|---|
| Components | % | mg/tablet | % | mg/tablet |
| (*R*)-zacopride hydrochloride | 3.45 | 3.53 | 27.51 | 70.60 |
| microcrystalline cellulose | 62.88 | 64.51 | 45.76 | 117.42 |
| mannitol | 25.54 | 26.21 | 18.59 | 47.70 |
| hydroxypropylmethylcellulose | 2.34 | 2.40 | 2.34 | 6.00 |
| sodium | 4.87 | 5.00 | 4.87 | 12.50 |
| carboxymethylcellulose | | | | |
| colloidal silicon dioxide | 0.19 | 0.20 | 0.19 | 0.50 |
| magnesium stearate | 0.73 | 0.75 | 0.73 | 1.88 |
| (Total) | 100 | 102.6 | 100 | 256.6 |

The first five components are sieved using a 63 µm grill and mixed for 3 minutes in a Lodigë 5 litre mixer granulator. Water is then added to the mixture (500 mL for 1 kg of dry powder) and the mixture granulated for 1 minute. The resulting granulate is dried at 50°C in a ventilated oven and calibrated at 800 µm. The remaining two components, Aerosil 200 and magnesium stearate are then added and mixed for 10 minutes in a Turbula^{®} mixer. The powder is then compressed using a Sviac PR12 tableting machine. In the case of tablets dosed at 3 mg (R)-zacopride base the tablets are of mass 102.6 mg containing 3.45 mg (*R*)-zacopride hydrochloride. In the case of tablets dosed at 60 mg (*R*)-zacopride base the tablets are of mass 256.6 mg containing 70.6 mg (*R*)-zacopride hydrochloride.

Intermediate doses are prepared by interpolation between these two formulae, varying the tablet mass and the percentage drug content.

## Claims

1. Use of (*R*)-zacopride or a pharmaceutically acceptable salt thereof for the manufacture of a medicament intended for the treatment of sleep apnea.

2. The use according to claim 1 wherein (*R*)-zacopride or a salt thereof is intended to administration at a daily dosage of up to 1 mg/kg of (*R*)-zacopride base.

3. The use according to claim 1 or 2 wherein the dosage amount of (*R*)-zacopride base is from about 3 to 60 mg per day.

4. The use according to claim 3 wherein the amount of (*R*)-zacopride base to be administered is from 10 to 30 mg.

5. The use according to any of claims 1 or 2 wherein (*R*)-zacopride or its salt is intended for administration by the oral, injectable, transdermal or rectal route.

6. The use according to claim 5 wherein (*R*)-zacopride or its salt is intended for oral administration.

7. The use according to anyone of claims 1 to 6 wherein the salt of (*R*)-zacopride is (*R*)-zacopride hydrochloride.
